Europäisches Patentamt

European Patent Office

Office européen des brevets

(19)

(11) Numéro de publication: **0 281 717**
**A1**

(12) **DEMANDE DE BREVET EUROPEEN**

(21) Numéro de dépôt: **87402813.7**

(51) Int. Cl.⁴ **A61B 3/10**

(22) Date de dépôt: **11.12.87**

| | |
|---|---|
| The title of the invention has been amended (Guidelines for Examination in the EPO, A-III, 7.3). | (71) Demandeur: **INSTITUT NATIONAL DE LA SANTE ET DE LA RECHERCHE MEDICALE (INSERM) (E.P.S.T.)**<br>**101, rue de Tolbiac**<br>**F-75013 Paris(FR)** |
| (30) Priorité: **11.12.86 FR 8617391** | |
| (43) Date de publication de la demande: **14.09.88 Bulletin 88/37** | (72) Inventeur: **Prablanc, Claude**<br>**42, rue du Professeur-Ranvier**<br>**F-69008 Lyon(FR)**<br>Inventeur: **Bailly, Gilles**<br>**18, rue Etienne Dolet**<br>**F-69600 Oullins(FR)**<br>Inventeur: **Pelisson, Denis**<br>**Le Logis Neuf Confrançon**<br>**F-01310 Polliat(FR)** |
| (84) Etats contractants désignés:<br>**AT BE CH DE ES GB GR IT LI LU NL SE** | (74) Mandataire: **Bugnon-Hays, Claudine**<br>**PATCO S.A. 10, rue Vivienne**<br>**F-75002 Paris(FR)** |

(54) **Procédé et dispositif de détection de la direction du regard par analyse du signal électro-oculographique.**

(57) La présente invention concerne une procédé de détection de la direction du regard consistant à déterminer l'orientation de la tête du sujet (1) et à traiter en temps réel le signal électrooculographique délivré par au moins une paire d'électrodes (2), (3) placées entre l'épithélium pigmentaire et la partie antérieure de la cornée, en vue de l'analyse de la position angulaire de l'oeil selon au moins un plan.

La présente invention concerne également un dispositif pour la mise en oeuvre de ce procédé, comportant un stimulateur oblique (5), un système de validation (28) de la fixation d'un point d'étalonnage du stimulateur (5), au moins une paire d'électrodes (2) et (3), et un système informatique de traitement des signaux.

EP 0 281 717 A1

FIG.1

## Procédé de détection de la direction du regard par analyse du signal électrooculographique et dispositif pour la mise en oeuvre dudit procédé.

La présente invention concerne un procédé de détection de la direction du regard consistant à déterminer l'orientation de la tête et à traiter en temps réel le signal électrooculographique recueilli par des électrodes placées entre les canthis externes en vue de l'analyse des mouvements oculaires selon au moins un plan, ainsi que le dispositif pour la mise en oeuvre dudit procédé.

Pour de nombreuses applications, la nécessité s'est imposée de disposer de techniques d'enregistrement fidèles et précises de la position de l'oeil. On peut citer des applications biomédicales en vue de la quantification des paramètres du système oculomoteur et des études ophtalmologiques, ou des applications dans le domaine de la commande de l'environnement par des handicapés moteurs, dans les domaines de la publicité ou des besoins militaires, et, plus généralement, toutes les fois où il est nécessaire de mesurer la direction du regard d'un sujet.

Parmi les procédés optoélectroniques mis en oeuvre actuellement, on distingue trois principes généraux de fonctionnement : la détection de la frontière iris-sclérotique, la captation du reflet cornéen et l'analyse vidéo simultanée de la pupille et du reflet cornéen.

La détection de la frontière iris-sclérotique nécessite une optique permettant de former sur un photorécepteur l'image de l'oeil ; la mesure s'effectue sur les deux changements de gradiant d'intensité lumineuse aux bords de l'iris. Bien que, théoriquement, il soit possible d'enregistrer les positions oculaires horizontales et verticales, dans la pratique ce système ne permet que l'enregistrement des mouvements oculaires horizontaux.

Cette technique nécessite, en outre, une immobilisation stricte du sujet par rapport au capteur optoelectronique. La résolution obtenue par ce procédé est de l'ordre de quelques minutes d'arc.

Le procédé de reflet cornéen consiste à recueillir la lumière d'un émetteur réfléchie par la cornée et captée sur deux photo-récepteurs disposés de façon symétrique devant l'oeil sur le plan horizontal.

Cette technique ne permet pas de dépasser 20 à 25 degrés de position oculaire. Comme pour la détection de la frontière iris-sclérotique, elle nécessite une bonne immobilisation des capteurs par rapport à la tête, et la précision se dégrade notablement dès que la position relative capteur-tête subit d'infimes variations.

On connaît également un procédé consistant en une analyse vidéo simultanée de la pupille et du reflet cornéen. Ce procédé comporte un traitement de signal compliqué qui aboutit au calcul du centre de gravité de la pupille, puis à la détermination du vecteur joignant ce centre de gravité au reflet cornéen.

La propriété de ce vecteur est de contenir les deux informations relatives à la position horizontale et à la position verticale de l'oeil. La restitution des coordonnées de l'oeil s'effectue à la fréquence de balayage de l'image, de l'ordre de 50 Hertz, ce qui interdit la reproduction de la dynamique des saccades oculaires.

De plus, si l'on veut s'affranchir d'une immobilisation de la tête par rapport à la caméra, il est nécessaire d'utiliser un système élctromagnétique d'asservissement de l'image de l'oeil au centre du photo-récepteur de la caméra. Ce système est d'un coût très élevé.

Du fait des contraintes et des limitations des procédés précédents, on a développé des procédés basés sur l'enregistrement du signal électrooculographique (e.o.g.). En raison de problèmes techniques tels que la polarisation des électrodes et des dérives permanentes des tensions d'offset, l'enregistrement du signal e.o.g. s'effectue généralement à travers des amplificateurs alternatifs à faible constante de temps. Cela interdit l'étude quantitative des mouvements oculaires lents et déforme la morphologie des saccades.

L'évolution dans la fabrication des électrodes a permis de réduire la dérive et d'enregistrer en continu les mouvements oculaires ; le signal e.o.g. étant remis à zéro lorsqu'il approche la zone de saturation de l'amplificateur.

La fidélité des enregistreurs e.o.g. de type connu n'est pas satisfaisante, en raison de la dérive persistante, des parasites externes, de la variabilité des gains du potentiel cornéen-rétinien et de la non-linéarité du signal en fonction de la position angulaire de l'oeil. De plus, les réglages mannuels des tensions d'offset et du gain entraînent des pertes de temps importantes.

Diverses améliorations ont été proposées dans l'état de la technique.

La demande de brevet français N° 85 04840 concerne un procédé de détermination stastistique de l'amplitude du potentiel e.o.g. Le signal e.o.g. passe à travers un filtre passe-bande. Un critère automatique de reproductibilité de la réponse à un stimulus répétitif permet d'évaluer la valeur du potentiel e.o.g. grâce à un moyennage sur trois formes consécutives cohérentes de la réponse.

Ce procédé a pour objet de s'affranchir des fausses réponses, des dérives de potentiel et des variations brutales du potentiel e.o.g. de façon automatique.

Il ne permet en aucune manière d'enregistrer en continu la position de l'oeil, et seules les excursions saccadiques rapides peuvent être mesurées, car le signal traverse un filtre passe-haut.

Le brevet américain N° 4 561 448 décrit un appareil d'enregistrement des signaux e.o.g. recueillis par deux paires d'électrodes, comportant des moyens d'annulation périodique de la tension d'offset. Ce dispositif ne permet pas le traitement des mouvements rapides du globe oculaire.

De plus, en raison des dérives de la tension d'offset, des variations des gains du signal e.o.g. et de la non-linéarité de la réponse des électrodes, la précision d'un tel appareil est limitée.

L'objet de la présente invention concerne un procédé de détection de la direction du regard, consistant à déterminer l'orientation de la tête du sujet et à traiter en temps réel le signal électrooculographique délivré par des électrodes placées entre les canthis externes et la partie extérieure de la cornée, en vue de l'analyse des mouvements oculaires selon au moins un plan, d'une grande fidélité et ne nécessitant pas d'intervention mannuelle.

Le procédé selon la présente invention consiste plus particulièrement en ce que:
- la tension de contre offset nécessaire pour annuler la tension d'offset du signal électrooculographique recueilli par les électrodes est déterminée au cours d'une phase de fixation par le sujet d'un point central d'un stimulateur, et en ce que la tension de contre offset est mémorisée,
- la dérive de la tension d'offset est compensée en temps réel par une tension de contre-offset déterminée par extrapolation des tensions de contre-offset déterminées au cours des précédentes phases de fixation du point central du stimulateur,
- le signal e.o.g. préamplifié et ainsi compensé est numérisé,
- un tableau de transfert entre les valeurs discrètes du signal e.o.g. digitalisé et les positions angulaires correspondantes de l'oeil, afin de permettre une indication en temps réel de la position vraie de l'oeil, est généré,
- ledit tableau de transfert est périodiquement réactualisé.

Le potentiel recueilli entre deux électrodes placées de part et d'autre de l'oeil, est d'autant plus important que la position angulaire de l'oeil est important. Ce signal brut est entaché d'erreurs et doit être transformé en temps réel pour représenter la direction vraie du regard, et ce, sans altération de la dynamique oculaire.

Le procédé sera décrit de façon plus précise pour des mouvements oculaires effectués dans le plan horizontal, mais il reste valide pour des mouvements verticaux et obliques.

La première étape du traitement consiste à éliminer les bruits parasites alternatifs. Le signal recueilli par les électrodes présente une amplitude de l'ordre de 5 à 20 microvolts par degré de position angulaire de l'oeil, et est, de ce fait, très sensible aux perturbations provoquées par des champs électriques. Ces champs électriques proviennent du réseau secteur présentant une fréquence de 50 Hertz, et des signaux électromyographiques résultant de l'activité involontaire des mâchoires et autres muscles faciaux, se mélant au potentiel e.o.g. par diffusion dans les tissus, et dont le spectre moyen est de 500 à 1 000 Hertz environ.

Ces signaux parasites peuvent être facilement éliminés de façon connue par un filtre passe-bas à coupure à faible pente, le fréquence de coupure étant environ égale à 80 Hertz, le spectre de fréquence des signaux e.o.g. étant essentiellement compris entre 0 et 40 Hertz.

S'il existe d'autres parasites de formes prédictibles, leur élimination est réalisé par superposition soustractive de la forme prédictive au signal perturbé.

La deuxième étape consiste à éliminer les signaux d'erreurs continus ou à très basse fréquence. Il s'agit des tensions d'offset recueillies entre les électrodes lorsque l'oeil est en position de repos, c'est-à-dire au centre de l'orbite. Cette tension d'offset est due à des polarisations légèrement différentes sur chaque électrode et à l'impédance des tissus en contact avec l'électrode. Cette tension d'offset peut être très supérieure à la valeur du signal utile, et atteint couramment 10 milivolts, soit 50 fois la valeur maximale du signal e.o.g.

Le principe de l'annulation de la tension d'offset repose sur l'utilisation d'un premier convertisseur de type analogique digital, réalisant la numérisation du signal délivré par le préamplificateur de signal e.o.g. fourni par les électrodes, et d'un second convertisseur de type digital analogique, transformant un signal de contre-offset délivré par un système de traitement informatique en un signal analogique réinjecté sur ledit amplificateur.

A l'instant initial de la période de la fixation fovéale d'un point central d'un stimulateur optique, le signal présent à l'entrée du préamplificateur correspond à la seule tension d'offset et provoque généralement la saturation de la chaîne d'amplification. Ce signal est amplifié et transmis sous forme numérique à un microordinateur.

Le microordinateur réinjecte une tension de contre-offset de même amplitude ramenée à l'entrée de la chaîne d'amplification.

Le microordinateur renouvelle cette opération de façon dichotonique de manière à faire converger la tension du signal amplifié correspondant à la position de repos de l'oeil vers zero.

La valeur de cette tension d'offset évolue lentement dans le temps.

Le procédé selon la présente invention consiste également à extrapoler la valeur de la tension d'offset entre deux périodes de réinitiation de la valeur de la tension d'offset, afin d'assurer la fidélité en temps réel du signal de sortie avec la position angulaire vraie de l'oeil.

Après compensation de la dérive d'offset, on constate que le potentiel e.o.g. est relié linéairement à la position angulaire de l'oeil par rapport à sa position "droit devant" jusqu'à une vingtaine de degrés. Au-delà, le potentiel e.o.g. amorce une saturation le rapprochant d'une courbe de type sinusoïdale.

Afin de linéariser le capteur, on effectue un étalonnage en demandant au sujet de fixer des cibles présentées à différentes excentricités. Le microordinateur gérant le procédé selon l'invention, construit une table de transfert des valeurs discrètes délivrables par le convertisseur analogique digital, afin de délivrer en temps réel la valeur vraie de la position angulaire.

L'étape suivante du procédé consiste à compenser la variation temporelle du gain du signal e.o.g., le gain étant proportionnel au rapport de la variation de la tension délivrée par les électrodes et de la variation correspondante du position oculaire à l'intérieur de la plage de linéarité, c'est-à-dire pour des positions angulaires inférieurs à une vingtaine de degrés. Ce gain peut varier de 10 à 50 % en un quart d'heure.

Dans le procédé selon l'invention, le gain e.o.g. est périodiquement réévalué au cours d'un phase de fixation par le sujet d'un ou deux points non centraux du stimulateur. Entre ces périodes de réévaluation, le microordinateur extrapole les variations de gain en temps réel, et réactualise le tableau de transfert.

La présente invention concerne également un procédé d'analyse des positions angulaires oculaires dans les deux plans vertical et horizontal, consistant à construire un tableau de transfert à deux dimensions, permettant de déterminer en temps réel le position angulaire oculaire dans le plan vertical et dans le plan horizontal, en fonction des tensions compensées et digitalisées délivrées par deux paires d'électrodes.

La présente invention concerne également un procédé de détection de la direction du regard, consistant à déterminer l'orientation de la tête du sujet sans contact mécanique, par projection sur une surface photosensible d'au moins une paire de sources de lumière, notamment infra-rouge, ponctuelles placées sur la tête du sujet, définissant un vecteur dont on peut calculer l'orientation par rapport à un vecteur fixe représentant une orientation "droit devant" de la tête.

Cette technique de mesure est insensible aux translations de faible amplitude de la tête, et ne code que la rotation par rapport à l'axe vertical. La surface photosensible peut avantageusement être constituée par un codeur analogique de coordonnées travaillant dans le spectre infra-rouge.

Selon une variante de la présente invention, l'étalonnage s'effectue en faisant fixer au sujet un point central pendant qu'il tourne la tête de plus ou moins 40 degrés autour de la position centrale. La compensation oculomotrice totale du mouvement de la tête lorsque celle-ci est en rotation à vitesse moyenne, permet d'acquérir les points d'étalonnage nécessaires à la construction du tableau de transfert.

La présente invention concene également un appareil pour la mise en oeuvre du procédé de détection de la position angulaire oculaire vraie en temps réel, comportant un stimulateur optique, un système de validation permettant au sujet de valider la fixation d'un point d'étalonnage , au moins une paire d'électrodes de recueil du signal e.o.g., au moins une chaîne d'amplification, des moyens de traitement du signal et un microordinateur gérant le procédé selon la présente invention.

L'invention sera mieux comprise, et d'autres buts, détails et avantages de celle-ci apparaîtront mieux à la lumière de la description explicative qui va suivre des différents modes de réalisation donnés à titre d'exemples non limitatifs, avec références et dessins non limitatifs annexés, dans lesquels :

- La figure 1 représente une présentation schématique du dispositif pour la mise en oeuvre du procédé selon l'invention.

- La figure 2 représente la courbe de tension e.o.g. en fonction de la position angulaire de l'oeil.

- La figure 3 représente de façon schématique la position des électrodes pour la détermination de la direction du regard selon les plans vertical et horizontal.

- La figure 4 représente les variations de la tension e.o.g. horizontale en fonction des positions angulaires oculaires dans les plans vertical et horizontal.

- La figure 5 représente les variations de la tension e.o.g. verticale en fonction des positions angulaires oculaires dans les plans vertical et horizontal.

- La figure 6 représente le schéma de principe du système de repérage du regard, tête libre.

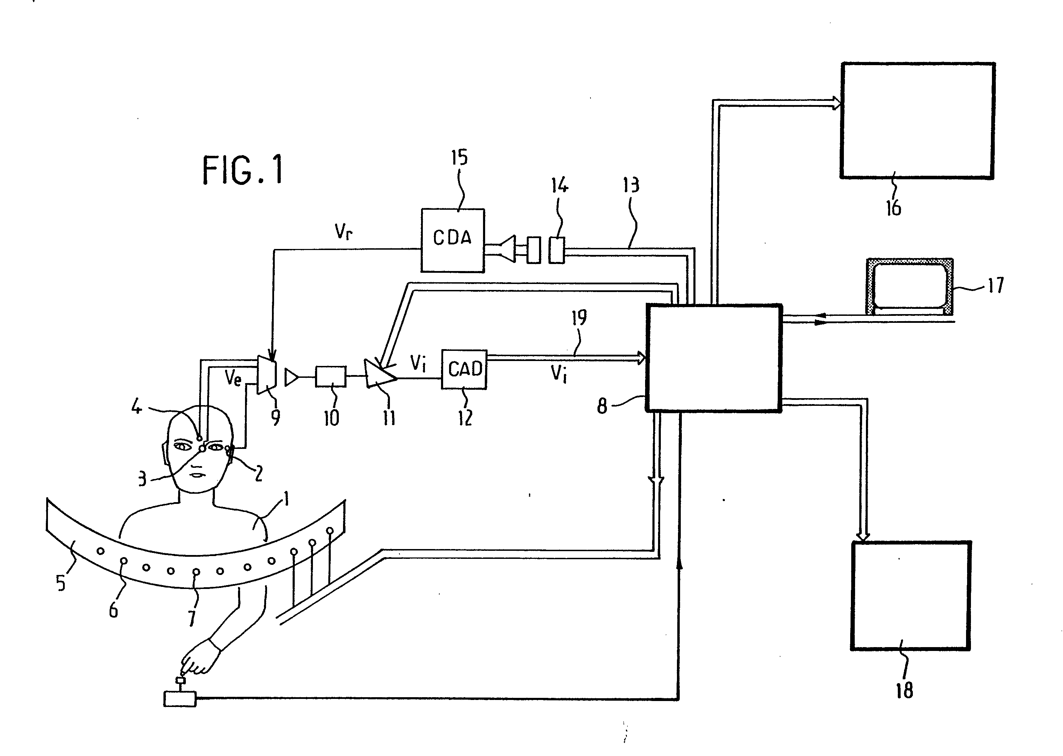

La figure 1 représente une vue schématique d'un dispositif destiné à la détermination de la direction du regard dans le plan horizontal d'un sujet (1).

La position de la tête est déterminée de façon connu, par exemple en la maintenant fixe, selon une orientation fixée.

4

On dispose entre les canthis externes deux électrodes (2), (3) destinées à recueillir le signal e.o.g., ainsi qu'une électrode de référence (4). Un stimulateur (5) comporte des points lumineux (6) et notamment un point central (7) dont l'allumage et l'extinction sont commandés par un microordinateur (8).

Le sujet (1) dispose, en outre, d'un bouton poussoir (28) lui permettant de valider la fixation des points d'étalonnage.

Le signal e.o.g. Ve recueilli par les électrodes (2), (3) a une amplitude de l'ordre de 5 à 10 microvolts par degré de position angulaire. Ce signal est préamplifié par un préamplificateur (9) isolé pour des raisons de sécurité par des amplificateurs d'isolement. Le gain du préamplificateur (9) est AG1. Le signal préamplifié est ensuite filtré par un filtre passe-bas (10) ayant une fréquence de coupure à 80 Hertz environ, et à pente faible (12 dB/octave). Ce filtre actif rejette également les bruits aléatoires, selon des techniques connues.

Le signal filtré est ensuite amplifié par un amplificateur (11) de gain AG2, délivrant un signal Vi. Ce signal Vi est échantillonné par un convertisseur analogique digital (12). La précision intrinsèque du signal e.o.g. ne dépassant pas 0,5 degré de position angulaire, un convertisseur analogique digital de 8 bits est suffisant pour coder la position de l'oeil entre ses positions extrêmes, soit environ plus ou moins 50 degrés, avec une précision de 50 : 128 = 0,4 degrés.

Au moment de l'étalonnage du système, le sujet fixe le point central (7) du stimulateur (5) et valide sa fixation par une action sur le bouton poussoir (28). Ce point central est constitué par un caractère qui ne peut être identifié que lorsqu'il est en fixation fovéale.

La tension Ve délivrée par les électrodes (2), (3) est alors égale à la tension d'offset. Cette tension provoque généralement la saturation du préamplificateur (9) et de l'amplificateur (10) et par conséquent de la tension image Vi. Cette tension Vi est numérisée par le CAD (12) et est transmise au microordinateur (8) qui réinjecte dans la boucle de contre-offset (13) une valeur numérique conduisant après passage à travers un opto-coupleur (14) et un convertisseur digital analogique (15) à une tension de contre-offset Vr.

A l'instant t1, la tension Vi est à nouveau numérisée et réinjectée dans la boucle de contre-offset. Le système renouvelle de façon dichotonique l'évaluation de la tension Vr permettant de faire converger la tension Vi correspondant à la fixation du point central (7) vers zéro.

Si on utilise un convertisseur digital analogique (15) de N bits, il est possible de compenser un offset égal à $0,5 \times 2^{(N-n)}$ fois l'excursion du signal utile, n étant le nombre de bits du CAD (12).

Avec un convertisseur CDA (15) de 16 bits et un convertisseur CAD (12) de 8 bits, il est possible de compenser une tension d'offset pouvant aller jusqu'à 128 fois la tension maximale utile.

La procédure de détermination de la valeur d'offset ne nécessite qu'une fraction de seconde, compte tenu du fait que la convergence de Vi vers 0 se fait en moins de N itérations dichotomiques.

La valeur de la tension d'offset est conservée en mémoire par le microordinateur (8).

La détermination de la valeur d'offset est effectuée périodiquement, afin de tenir compte de sa dérive parfois importante. Entre deux périodes de détermination, le microordinateur recalcule en temps réel la valeur de la tension Vr à réinjecter dans la boucle de contre-offset (13) par une extrapolation polynomiale, basée sur les valeurs mémorisées au cours des détermination précédentes.

L'intervalle de temps séparant deux déterminations est avantageusement asservi à la différence entre la valeur d'offset effectivement déterminée et la valeur extrapolée. L'intervalle de temps entre deux déterminations peut être sensiblement inversement proportionnel à la différence entre la valeur vraie de la tension d'offset et la valeur extrapolée.

Il est possible ainsi d'augmenter le rapport :

$$\frac{\text{temps d'étude de la direction du regard}}{\text{temps d'étalonnage}}$$

et d'espacer les périodes où le sujet (1) doit refixer le point central (7).

Le microordinateur gère également une procédure d'établissement du tableau de transfert entre le signal Vi numérisé et la position angulaire vraie.

Pour cela on effectue un étalonnage sur plusieurs points situés à droite et à gauche du point central (7) du stimulateur (5). A l'aide de ces mesures, le système recherche la fonction analytique qui s'ajuste aux points d'étalonnage selon un critère de minimisation quadratique. Le système mémorise cette fonction sous forme d'un tableau numérique de n valeurs discrétes correspondant aux excursions maximales du signal Vi, n étant le nombre de bits du convertisseur CAD (12). Ce tableau permet d'établir la concordance en temps réel entre le signal Vi et la position angulaire oculaire pour chaque échantillonnage à la cadence de 400 Hertz. Cette fréquence d'échantillonnage à 400 Hertz a été choisie car elle représente un multiple de la fréquence secteur et facilite la mise en oeuvre d'un filtre logiciel réjecteur des parasites secteur.

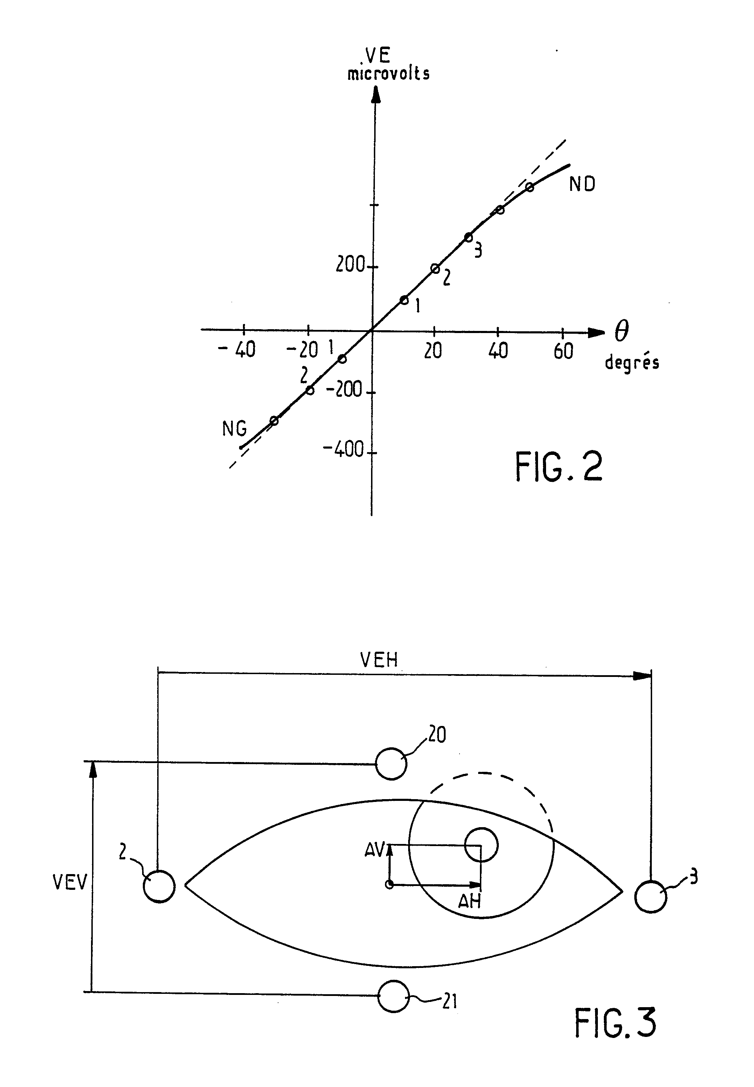

La figure 2 représente un exemple de courbe représentant la valeur de la tension Ve en fonction de la position angulaire oculaire. La pente de cette courbe dans la zone de linéarité représente le gain e.o.g. Ce gain évolue dans le temps, et le maintien de la fidélité du capteur exige une correction permanente.

L'étude des propriétés de la dérive du gain e.o.g. a montré qu'une hypothèse de dérive isomorphe conduit à une précision suffisante.

Le dispositif selon la présente invention mémorise un coéfficient d'homothétie aux moments de l'établissement du tableau de transfert, et recalcule entre lesdits moments chaque point du tableau en fonction du nouveau coéfficient d'homothétie. Le dispositif permet de cette façon de déterminer avec une grade fidélité la valeur vraie de la position angulaire oculaire, en temps réel et à la cadence d'échantillonnage. Cette information peut être présentée sous forme graphique sur une table traçante (16) ou un écran vidéo (17) ou restitué sous forme numérique après mémorisation et analyse par le microordinateur (8) à l'aide d'une imprimante (18).

Le dispositif comporte, en outre, des moyens permettant de tester de façon permanente l'impédance des électrodes, afin de détecter une quelconque anomalie.

Selon une variante, la correction de la variation du gain du signal e.o.g. par recalcul du tableau de transfert est complétéé par une sélection automatique du gain de l'amplificateur (11) par un signal de commande généré par le microordinateur (8) lorsque le coéfficient d'homothétie devient supérieur à une valeur limite fixée en fonction des caractéristiques dudit amplificateur (11). Ces signal de commande est transmis à l'amplificateur (11) par le circuit de contre-réaction(19).

Le microordinateur (8) est commandé par un programme informatique réalisant notamment les fonctions suivantes :

```
┌─────────────────────────────────────────┐
│  Test de l'impédance des électrodes      │
└─────────────────────────────────────────┘
                    │
   ╭───╮            ▼
   │ α │────────────────────────────────┐
   ╰───╯                                 │
                                         ▼
        ┌─────────────────────────────────────────┐
        │  Mise en fonction du point central (7)   │
        │         du stimulateur (5)               │
        └─────────────────────────────────────────┘
```

NON

Validation par le sujet (1)
de la fixation du point central (7) ?

OUI

Vi = 0 ?

OUI

NON

Réinjection d'une tension de contre-offset
d'une valeur augmentée de Vi

Mémorisation de la tension d'offset Vr0

Mise en fonction des points lateraux
extrêmes du stimulateur (5)

NON

Validation par le sujet ?

OUI

Mémorisation de Vi1

Mise en fonction d'un autre point latéral du stimulateur (5)

NON

Validation par le sujet ?

OUI

Mémorisation de Vi2, calcul du gain optimum de l'amplificateur (11)

Mise en fonction du point central (7)

NON

Validation par le sujet (1) de la fixation du point central (7) ?

OUI

Vi = 0 ?    OUI

NON

Réinjection d'une tension de contre-offset d'une valeur augmentée de Vi

8

```
                                                                          OUI
        ┌─────────────────────────────────┐ ◄──────────┘
        │ Initialisation d'un indice "a"  │
        └─────────────────────────────────┘
                        │
                        ▼
                 ┌──────────────┐
                 │  a = a + 1   │
                 └──────────────┘
                        │
        NON             ▼
        ┌──► ┌─────────────────────────────────┐
        │    │ Mise en fonction d'un point a   │
        │    │      du stimulateur (5)         │
        │    └─────────────────────────────────┘
        │                    │
        │   NON              ▼
        │    ┌───────────────┐
        │    │               ╲
        │    └──────►     ╱Validation ?╲
        │            ╲               ╱
        │                 ╲       ╱
        │                     │ OUI
        │                     ▼
        │         ┌───────────────────────┐
        │         │   Mémorisation de Va  │
        │         └───────────────────────┘
        │                     │
        │                     ▼
        │         ╱                         ╲
        └──── ╱ a = valeur maximale préfixée ? ╲
              ╲                               ╱
                 ╲                         ╱
                        │ OUI
                        ▼
    ┌───────────────────────────────────────────┐
    │ Etablissement du tableau de transfert par │
    │   extrapolation polynomiale à partir des  │
    │      a couples (Va, position angulaire     │
    │      du point a sur le stimulateur (5))    │
    └───────────────────────────────────────────┘
                        │
                        ▼
    ┌───────────────────────────────────────────┐
    │ Réévaluation de la tension de contre-offset│
    └───────────────────────────────────────────┘
                        │
                        ▼
```

```
┌─────────────────────────────────────────────────┐
│  Détermination de la fonction d'évolution        │
│       de la tension de contre-offset             │
└─────────────────────────────────────────────────┘
```

```
┌─────────────────────────────────────────────────┐
│  Fixation du temps T prévu entre 2 périodes      │
│       de réévaluation des tensions offset        │
│  et de la fonction de transfert, en fonction     │
│  de la cohérence entre les valeurs prévues       │
│         et les valeurs mesurées,                 │
│       initialisation du temps écoulé t           │
└─────────────────────────────────────────────────┘
```

OUI

```
┌─────────────────────────────────────────────────┐
│  Mesure de la position angulaire de l'oeil,      │
│     sortie en temps réel des valeurs vraies      │
│  telles que résultant de la compilation du signal│
│   e.o.g. corrigé avec le tableau de transfert,   │
│    mémorisation pour étude en temps différé      │
└─────────────────────────────────────────────────┘
```

NON

t = T ?

OUI

```
┌─────────────────────────────┐
│   Réévaluation rapide,       │
│   élimination des artefacts  │
└─────────────────────────────┘
```

Cohérence entre les valeurs prévues
et les valeurs réévaluées ?

α

NON

Pour détecter la position angulaire de l'oeil dans un plan horizontal et vertical, en dispose deux paires d'électrodes (2), (3) et (20), (21) telles que représentées sur la figure 3. La composante verticale du signal e.o.g. Vev est recueillie entre les électrodes (20) et (21). A chaque position de l'oeil (Ah, Av) est associé un couple de mesures (Veh, Vev). Les signaux horizontaux et verticaux e.o.g. sont corrigés comme dans le cas de la détermination de la position angulaire selon un seul plan.

Pour la construction du tableau de transfert, les même méthodes d'ajustement par fonction analytique sont appliquées. Après fixation de N points d'un stimulateur (5) à 2 dimensions, on obtient N courbes de niveau Vh (Ah) et N courbes de niveau Vv (Av). Les courbes de niveau adjacentes sont divisées en deux, afin de construire un réseau de courbes intermédiaires plus serrées, en faisant l'hypothèse d'une variation linéaire entre deux courbes.

La subdivision est poursuivie jusqu'à la limite de précision intrinsèque de signal e.o.g., soit environ 0,5 degré.

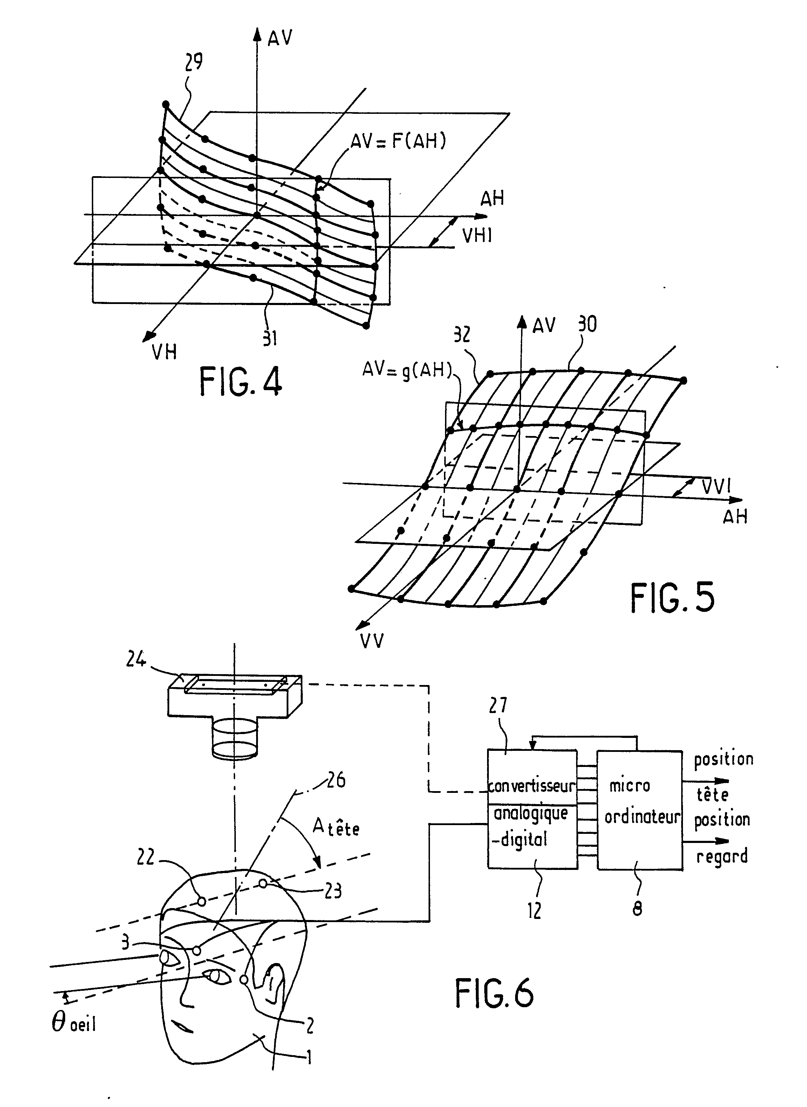

On obtient ainsi un premier réseau de courbes représenté en figure 4, dont l'enveloppe (29) représente la fonction Vh (Ah, Av) pour le signal e.o.g. horizontal, et un second réseau de courbes, représenté en figure 5, dont l'enveloppe (30) représente la fonction Vv (Av, Ah) pour le signal e.o.g. vertical.

On construit alors un réseau de courbes orthonormées aux courbes de niveau Vh (Ah) (31) sur l'enveloppe (29) représentant la fonction Vh (Av, Ah), puis un deuxième réseau de courbes orthonormées aux courbes de niveau Vv (Av) (32) sur la surface (30) représentant la fonction Vv (Av, Ah). Ces réseaux sont définis avec un incrément égal au nombre de bits de définition de chacun des signaux e.o.g. Vh et Vv. Le nombre maximum d'informations définies par les mesures Vh et Vv, si chacun des convertisseurs analogiques digitaux possède n bits, est $(2^n)^2$ points. On calcule pour chacun de ces points l'intersection des courbes Av = f (Ah) pour Vhi constante, et Av = g (Ah) pour Vhj constante, ce qui définit un couple de position de l'oeil Ahi, Avi correspondant au couple Vhi, Vvj. On dispose ainsi d'un tableau de transfert de dimension $(2^n)^2$, et à tout couple de measure Vh, Vv correspond ainsi directement par lecture dans le tableau un couple Ah, Av de position angulaire de l'oeil.

Selon une variante, le dispositif restitue en temps réel les positions angulaires exactes du regard à partir, d'une part, du signal e.o.g. recueilli par des électrodes de surface périorbitales jetables et traité selon le procédé de l'invention, et, d'autre part, d'un signal optoélectronique codant la seule rotation de la tête.

Un tel dispositif élimine toute limitation du champ visuel, ainsi que toute contrainte mécanique de la mobilité de la tête.

Le sujet (1), tel que représenté en figure 6, porte sur la tête deux sources lumineuses ponctuelles (22), (23), par exemple des diodes électroluminescentes émettant dans le proche infra-rouge. La projection de ces deux points sur une surface photosensible (24) définit un vecteur permettant de calculer l'orientation (25) de la tête par rapport à une direction de référence (26) définissant la position de repos "droit devant". Cette mesure de l'orientation (25) est indépendante d'une translation faible de la tête du sujet (1). La surface photosensible peut être avantageusement constituée par un codeur analogique de coordonnées X, Y, travaillant dans le spectre infra-rouge.

Le temps de mesure d'un couple de coordonnées par un tel type de capteur est de l'ordre de 25 microsecondes. A partir des couples (X1, Y1), correspondant à l'orientation de référence (26), et (X2, Y2), correspondant à l'orientation instantanée de la tête, on calcule la tangente de l'angle de rotation de la tête A :

$$\text{tangente } A = \frac{X2 - X1}{Y2 - Y1}$$

Il suffit alors de pointer directement la valeur de tangente A dans un tableau précalculé des arcs tangentes pour obtenir en temps réel l'orientation de la tête.

Selon un mode de réalisation avantageux, l'étalonnage, c'est-à-dire la construction de tableau de transfert, s'effectue en faisant fixer un point central (7) par le sujet, tandis que sa tête tourne d'un angle d'environ plus ou moins 40 degrés avec une vitesse de rotation moyenne.

Du fait des propriétés physiologiques du système vestibulo-oculaire permettant la compensation oculomotrice totale du mouvement de la tête, l'angle relevé par la surface photosensible (24) et transmis par un convertisseur analogique digital (27) sous forme numérique au microordinateur (8) correspond exactement à la position angulaire orbitaire de l'oeil fixant le point central (7) du stimulateur (5).

On réalise ainsi l'acquisition de nombreux couples (position angulaire vraie de l'oeil, valeur du signal e.o.g. recueilli pa les électrodes (2), (3)) permettant de régler le gain e.o.g. et de construire le tableau de transfert.

La mise en oeuvre de cette variante peut se résumer ainsi :

- Pose des électrodes jetables sur les canthi externes du sujet (1). Le test d'impédance indique notamment si la pose est défectueuse.

- Pose de deux diodes infra-rouges sur les oreilles du sujet (1) et positionnement naturel du sujet selon l'orientation de référence.

- Fixation par le sujet (1) du point central (7) du stimulateur (5) et validation par appui sur le bouton poussoir (28). Le microordinateur (8) ramène alors automatiquement la tension d'offset à zéro selon la procédure déjà décrite.

- Le sujet fixe ensuite les points périphériques extrêmes qu'il aura à explorer, ce qui permet une sélection automatique du gain de la chaîne d'enregistrement.

- Le sujet tourne volontairement la tête dans le plan horizontal, de plus ou moins 40 degrés, tout en maintenant la fixation sur le point central (7). L'opérateur dispose sur l'écran du tracé de la position angulaire de l'oeil. Si aucune erreur de fixation ne s'est produite, il valide l'étalonnage.

- A des intervalles T déterminés par la cohérence des précédentes estimations, un signal sonore prévient le sujet de la nécessité d'une réévaluation de la tension offset et du tableau de transfert.

Selon une variante, on dispose sur la tête du sujet deux paires de sources lumineuses ponctuelles afin de déterminer l'orientation de la tête dans un plan horizontal et dans un plan vertical.

Selon une autre variante, l'orientation de la tête dans le plan vertical est déterminée par la longueur et la direction du vecteur déterminé par la projection des sources lumineuses ponctuelles (22), (23).

La présente invention n'est pas limitée aux exemples et modes de réalisation décrits ci-dessus, mais s'étend à toutes les variantes envisageables.

## Revendications

1) Procédé de détection de la direction du regard consistant à déterminer l'orientation de la tête du sujet (1) et à traiter en temps réel le signal électrooculographique délivré par au moins une paire d'électrodes (2), (3) placées entre les canthis externes en vue de l'analyse de la position angulaire de l'oeil selon au moins un plan, caractérisé en ce que :

- la tension de contre-offset, nécessaire pour annuler la tension d'offset du signal électrooculographique recueilli par lesdites électrodes (2), (3) et préamplifié par un préamplificateur (9), est déterminée au cours d'un période de fixation par le sujet (1) d'un point central (7) d'un stimulateur (5) et est mémorisée ;

- la dérive de la tension d'offset est compensée en temps réel par une tension de contre-offset déterminée par extrapolation des tensions de contre-offset déterminées au cours des précédentes périodes de fixation du point central (7) du stimulateur (5) ;

- le signal électrooculographique ainsi compensé est mémorisé;

- un tableau de transfert entre la valeur discrète du signal électrooculographique digitalisé et la position angulaire vraie de l'oeil est généré ;

- ledit tableau de transfert est réactualisé périodiquement.

2) Procédé selon la revendication 1, caractérisé en ce que la tension d'offset est compensée par une tension de contre-offset déterminée par une injection itérative d'une tension égale à la somme de la tension de contre-offset précédemment évaluée et de la tension délivrée par les électrodes (2), (3) au cours de la période de fixation du point central (7) jusqu'à annulation du système du signal délivré par un convertisseur analogique digital convertissant le signal électrooculographique amplifié et compensé.

3) Procédé selon l'une quelconque des revendications 1 ou 2, caractérisé en ce que la tension de contre-offset nécessaire à la compensation de la tension d'offset est réévaluée entre chaque détection de la position angulaire de l'oeil par extrapolation polynomiale à partir des valeurs de contre-offset précédemment déterminées.

4) Procédé de détection de la direction du regard dans le plan vertical et dans le plan horizontal selon l'une quelconque des revendications 1 à 3, caractérisé en ce que l'on construit un tableau de transfert à deux dimensions entre les valeurs électrooculographiques compensées digitalisées, délivrées par deux paires d'électrodes, et la position angulaire vraie de l'oeil.

5) Procédé de détermination de la direction du regard selon l'une quelconque des revendications 1 à 4, caractérisé en ce que l'orientation de la tête par rapport à une direction de référence est déterminée par un vecteur obtenu par projection d'une paire de sources lumineuses ponctuelles solidaires de la tête dudit sujet sur une surface photosensible restituant un signal représentant les coordonnées dudit vecteur.

12

6) Procédé de détermination de la direction du regard selon l'une quelconque des revendications 1 à 5, caractérisé en ce que la détermination du tableau de transfert, et de l'évolution dans le temps du tableau de transfert, est réalisée à partir d'une série de couples (valeur du signal électrooculographique, position angulaire vraie de l'oeil) acquis par simple fixation d'un point central (7) du stimulateur (5) pendant la rotation de la tête autour d'un axe vertical.

7) Dispositif pour la mise en oeuvre du procédé de détermination en temps réel de la direction du regard selon l'une quelconque des revendications 1 à 6, caractérisé en ce qu'il comporte un stimulateur optique (5), un système de validation de la fixation d'un point d'étalonnage dudit stimulateur (5), au moins une paire d'électrodes (2), (3) et un système informatique de traitement des signaux.

8) Dispositif pour la détermination de la direction du regard selon la revendication 7, caractérisé en ce qu'il comporte une paire de sources lumineuse ponctuelles, destinées à être disposées sur la tête du sujet, et un capteur photosensible.

FIG.1

0 281 717

FIG.2

FIG.3

FIG. 4

FIG. 5

FIG. 6

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | GB-A-2 170 910  (THE MARCONI CO. LTD) <br> * Figures 3-6; page 2, ligne 48 - page 5, ligne 43 * <br> --- | 1,2,5,7,8 | A 61 B    3/10 |
| A | DE-A-2 828 532  (HOFFERBERTH) <br> * Figures 1-3; page 10, ligne 10 - page 16, ligne 14 * <br> --- | 1,7 | |
| A | DE-A-2 709 131  (TÄUMER) <br> * Figure 6; revendication 1; page 17, ligne 28 - page 18, ligne 15 * <br> --- | 1,2 | |
| A | IEEE TRANSACTIONS ON BIO-MEDICAL ENGINEERING, vol. 22, no. 5, mai 1975, pages 427-428, New York, US; R.L. MASON: "An automatic zeroing circuit" <br> --- | 1,2 | |
| D,A | US-A-4 561 448  (BUCHAS) <br> ----- | | |

DOMAINES TECHNIQUES RECHERCHES (Int. Cl.4)

A 61 B

Le présent rapport a été établi pour toutes les revendications

| Lieu de la recherche | Date d'achèvement de la recherche | Examinateur |
|---|---|---|
| LA HAYE | 18-03-1988 | CHEN A.H. |